# EUROPEAN PATENT APPLICATION

(11) **EP 2 246 324 A1**
(43) Date of publication of application: **03.11.2010**
(21) Application number: 09425147.7
(22) Date of filing: 21.04.2009
(51) Int. Cl.: C07C 309/10, C07C 309/14, C11D 1/12, C11D 1/90, A62D 1/00

(54) **Sulphonic function fluorine compounds and their use**

(71) Applicant: MAFLON S.R.L., 24060 Castelli Calepio (IT)
(72) Inventor: Maniero, Francesco, 25030 Paratico, Brescia (IT); Caporiccio, Gerardo, 20149 Milano (IT); Bevilacqua, Marco, 36078 Valdagno, Vicenza (IT)
(74) Representative: Pulieri, Gianluca Antonio

(57) **Abstract**

The present invention relates to compounds containing a fluoro hydrocarbon terminal and a sulphonic function terminal obtained by a series of chaining reactions of a perfluorocarbon segment with elevated hydrophobic and oleophobic powers with an aliphatic segment containing heteroatoms (O, N, S), in turn terminated by a hydrophilic sulphonic group in the form of a free acid or salt with an alkaline or ammonium ion.

Such compounds also have excellent amphipatic properties in highly saline aqueous environments.

The present invention relates further to the use of the compounds described.

## Description

The present invention relates to a sulphonic function compound and its use.

### STATE OF THE ART.

The first industrial production process of perfluorinated acids was performed by electrochemical fluorination (ECF) of the fluorines of the corresponding aliphatic acids: in the case of carboxy acids a mixture of products with a branched and linear chain composed of about 70% of carboxy fluorines and 30% of inert perfluoroalkanes also containing oxy-carbocyclics was obtained.

The perfluorocarboxy and sulphonic acids have the characteristics of strong acids, the components with six, eight or more carbon atoms in particular are used in commercial applications, pure or mixed, on account of their excellent surfactant properties superior to their hydrocarbon homologues.

The electrochemical fluorination process has also been used to obtain fluorides of the perfluoroalkanesulphonic acids.

The derivatives of perfluorooctanoic acid (PFOA) and perfluorooctansulphonic acid (PFOS) are particularly widespread in applications, as agents or intermediates able to confer characteristics of low surface tension, filmogenous properties with a high covering capacity, foam regulation and a repellent effect of inorganic or organic contaminants such as for example organic or inorganic dirt stains; these properties can be transferred to other systems when the aforesaid compounds are added to aqueous formulations or to organic solvents of paints, varnishes and protective compositions for surfaces of solid materials such as metals, wood, natural and synthetic polymers, or when applied to woven or nonwoven materials made from vegetable or animal fibre such as papers, fabrics and leathers.

In addition many derivatives of the PFOA and PFOS, obtained by means of the metathesis of the acid radical (carboxy or sulphonic) to functional groups such as the alkyl amide or alcohol amide groups, have subsequently been transformed into acrylates, meta acrylates and polymerizates.

Such mono-molecular or polymeric products have also been used as basic compounds or additives to paints, varnishes and inks - carried in an organic or mixed aqueous medium - in as much as able to confer special and typical surface properties, of wettablity and filmogenous properties to compositions of protective and repellent coatings for paper and cardboard, leathers and fabrics with a high covering capacity and absence of surface defects compared to inert or aggressive agents carried in an aqueous or organic medium; said monomolecular or polymeric products are also utilised as additives able to formulate repellent and protective compositions for clothing and furnishing fabrics, carpets, drapes; in addition they are also used in formulas for other sectors such as the photographic sector, or for water-based fire extinguishing foams.

The favourable commercial development of such products and specialities has provoked interest in the creation of a method of synthesis of perfluorocarbon segments alternative to electrochemical fluorination: the most interesting alternative process, transposed to an industrial scale, has been found in the radicalic telomerisation of tetrafluoroethylene using perfluoro iodate telogens, as has been developed by Du Pont, Atochem (now Archema) and Daikin (US 3,226,449; US 5,268,516; US 5,650,565; EP 1,364,934).

C2F5I has been used to produce linear iodoperfluoroalkanes F (C2F4)k-I, of various molecular weight (MW), containing from 2 to 6-8 units of TFE; up until the end of the nineties telomers with 3-6 units of TFE were used, those with 4-5 units of TFE being preferred. The iodated telomers were transformed by metathesis using the intermediate Rf-CH2CH2-I in alcohols and amines, the most interesting intermediates having structures of the type: Rf-CH2CH2-OH, Rf-CH2CH2-NH2, where Rf is the linear perfluorocarbon radical, deriving from telomerisation of the TFE.

From the respective amides and ethers of the acrylic and metacrylic acids the corresponding fluorinated polymers and copolymers used to formulate paints and varnishes dispersed in organic or aqueous mediums were obtained; fluorinated acids and the corresponding derivative amides, amide-alcohols, finding the same applications as the analogous compounds obtained by electrochemical fluorination, were also obtained.

In the year 2000 the company 3M, manufacturer of PFOA and PFOS products using ECF announced their voluntary decision to abandon production of PFOA and PFOS acids by means of ECF after discovering the occurrence of illness in workers involved in the synthesis and transformation processes and finding traces of the compounds in the blood and liver of some of these people.

In actual fact, already back in the '80s the EPA had started inspections of the ground and ground water in the areas where said products were produced and formulated, so as to verify the effective degree of degradation performed by the natural gents and microorganisms in the ground on the fluorinated (FOC) and perfluorinated (PFC) organic compounds (PFC); it was found that - while the partially fluorinated organic compounds were degraded in the bonds of the functional group - many FOC like PFC were stable and among these fifty or so PFOA and PFOS and some of their derivatives were identified which were particularly resistant and able to migrate a significant distance from the site of production and transformation, on account of their insolubility and chemical and physical stability, as well as their low volatility.

The mechanism which favours the elevated migratability of such compounds has not yet been fully explained, especially for some derivatives with particularly low solubility and volatility, also in relation to the PFOA and PFOS precursors; the most logical explanation seems to depend on the food chain which numerous individuals of the mineral, vegetable and animal species participate in.

Specifically, the presence of PFOS and of some specific derivatives - dispersed in nature and found in remote areas - was at higher concentrations than that of PFOA, as was found in the blood of animals living in the Great American Lakes, in the Northern Arctic and Alaskan seas and in the northern Pacific ocean.

The difficulty of controlling the diffusion of such products in the environment as well as in the production sites of the precursors and formulations, where strict controls are foreseen for the elimination of scrap and waste, - while not underestimating the failure of operators to adopt protective measures during routine hygiene procedures and during normal behaviour - is more critical in the sites and circumstances of use by unaware consumers in houses and offices, especially in emergency situations such as fire-fighting in open air areas such as airports, ports, military bases etc. [cfr. Environm. Sci. & Technology, 147(4), 2002].

The usual surfactants composed of hydrocarbon compounds for domestic and industrial use end up with waste at the waste processing plants, the effluents from which drain into the groundwater and natural cumulative deposits; they undergo various processes depending on the structure: cationic and non-ionic surfactants are absorbed by the ground and some are degraded by microorganisms in the environment at various speeds, while the linear alkylbenzenesulphonates (ALS) are more persistent in anaerobic conditions but fully degraded in aerobic conditions.

As regards toxicity, this decreases from the amine cationic surfactants to the quaternary ammonium salts, non-ionic and anionic surfactants which have least toxicity, and also as the length of the chain decreases.

In fact, tracking tests performed on rats which had been administered - both orally and intravenously - doses of anionic surfactants type ALS (marked 14C) showed that within 24 hours these had been completely eliminated in the urine and faeces and that the metabolic compounds were products of oxidation and molecular degradation [S. Inoue, Toxicology Sci. J. 1982, 130; G-G. Ying, Environ. International 2006, 32 (3) 417; M. A. Lewis, Water Res. Watrag, 1991, 25 (1) 101; D.J. Versteeg, Environ. Toxicology,1997 ,16 (5) 1051; J. Jensen, Ecotoxicology & Environ. Safety, 1999, 93; M. Hampel, ibid. 2002, 53].

The EPA decided to sponsor further investigations into the ecotoxicity of the surfactants derived from PFOA, aimed at gathering information for a motivated decision and issued a recommendation for a voluntary reduction of 95% of emissions of PFOA by 2010 with a long term goal of eliminating PFOA in consumer products entirely by 2015 [C. M. Stevenson, Environ. Sci. Technol. 2006, 40, 5590].

The present invention therefore sets out to overcome the drawbacks of the known technique and specifically the aforesaid.

Such drawbacks are overcome by compounds according to claim 1, by means of the synthesis of such compound according to claim 8 and by means of the applications according to claims 9 to 15. The dependent claims illustrate preferred embodiment variations.

### DESCRIPTION OF THE INVENTION.

The present invention describes a method for preparing a new class of compounds with specific surfactant properties and having a structure formed of a polyfluorinated aliphatic chain also containing in the central segment a group with hydrophilic characteristics and one or more heteroatoms (Oxygen, Nitrogen, Sulphur) one of which is bound to a hydrocarbon segment formed of 2 to 6 atoms of carbon and terminated by a sulphonic functional group.

The fluorinated sulphonic surfactants may be anionic and be in the form of a free or salified sulphonic acid, like the derivatives of sulphonic acid and its alkaline or ammonium salts; or they may be betainic, when a quaternary ammonium group is present in which case the positive charge of the quaternary ammonium group is neutralised by the negative charge of the sulphonic anion. The overall neutral charge is produced by the presence of two charges of opposite signs existing in a non-adjacent position.

More specifically the typical structure of the new class of surfactants according to the invention has at the head end, an R' hydro-oleophobic group, formed of a segment of perfluorocarbon chain composed of 2 to 6 atoms of carbon, preferably from 3 to 6, even more from 4 to 6, R' may be an Rf-Rh group where Rh, when present, is an alkylenic group composed of two, three and even more atoms of carbon (in one embodiment even 11, Rh is bound by a C-C bond to Rf and acts as a spacer between Rf and the rest of the molecule of surfactant; the R' hydro-oleophobic group is connected by a C-C bond or a bond between carbon and an ethereal oxygen to a structural L-Q grouping containing heteroatoms among which ethereal oxygen, amine or ammonium nitrogen and/or other substituents such as the oxyhydril or amino groups which contribute to conferring hydrophilic properties to the L-Q grouping which is lastly terminated by a sulphonic acid group or sulphonic group; specifically, according to one embodiment of the invention the Rf group is directly bound to a bivalent radical 2-hydroxy-1,3-propylene (L), and these to an amine or ammonium alkylene sulphonic or, as an alternative option, the Rf-Rh group is bound to an oxy(alkyleneoxy)alkylene sulphonic radical.

Some surfactants obtained according to the invention have the structures illustrated below, shown by way of non-limiting examples:
- I - betainic derivatives 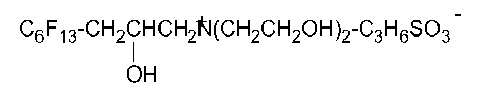
- II - betainic derivatives 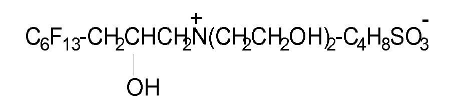
- III - betainic derivatives 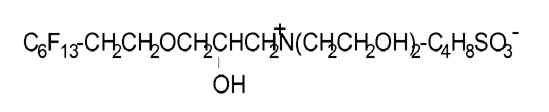
- IV - anionic derivatives in basic environment 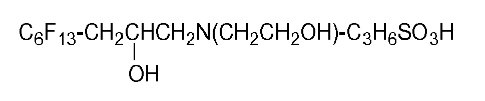
- V - anionic derivatives in basic environment 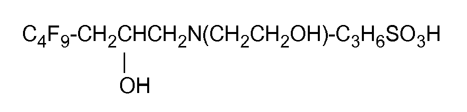
- VI - betainic derivatives

   C₆F₁₃CH₂CH (OH) CH2N⁺ (CH₃) (CH₂CH₂OH)-C₃H₆SO₃
- VII - betainic derivatives

   C₆F₁₃CH₂CH (OH) CH2N⁺ (CH₃) (CH₂CH₂OH)-C₄H₈SO₃⁻
- VIII - anionic derivatives in basic environment

   C₆F₁₃ (CH₂) 11OC4H8SO3H;
- VIII^{I} - betainic derivatives

   CₙF₂ₙ₊₁CH₂CH₂OC₂H₄N⁺ (CH₂CH₃)₂-C₄H₈SO₃⁻
- VIII^{II} - anionic derivatives in basic environment

   CnF₂n⁺₁-CH₂CH₂OC₄H₈-O-C₃H₆SO₃H
- VIII^{III} - anionic derivatives in basic environment

   CnF₂n⁺₁-CH₂CH₂CH₂O(C₂H₄O)₇O-C₃H₆SO₃H
- VIII^{IV} - betainic derivatives

   CnF₂n⁺₁-CH₂CH₂OC₂H₄-N⁺(C₂H₅)₂-C₄H₈SO₃⁻
- IX - betainic derivatives

   C4F9-C2H4-N⁺-(C2H40H) 2-C4H8-SO3-

   - X - betainic derivatives

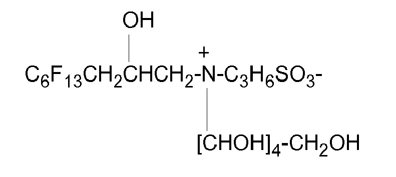

The representative structure of the invention may be described by the following general formula (I):

R'-L-Q-S (I)

wherein:
- R' is the hydro-oleophobic group, formed of a perfluorinated Rf radical or of an Rf-Rh sequence; Rh when present acts as spacer between Rf and the L group;
- L is composed, as a non-limiting example, of 1-oxy-2-hydroxypropylene (-OCH2CH (OH) CH2-), 2-hydroxypropylene groups or oxyalkylene, such as oxyethylene, oxybutylene, oxy-poly (ethylenoxy); a polyethereal segment (C2H4-O)p-(C3H6O)q- where p ranges from 1 to 10, preferably 1 to 6; q ranges from zero to 6;
- Q-S is a grouping type:
   a) -O-CnH2n-SO3H, where -O- is an atom of ethereal oxygen and where the sulphonic group may be in an acid form or its alkaline salt and the CnH2n group is linear or branched and composed of two to 8 atoms of carbon, preferably of three to four atoms of carbon;
   b) -S-CnH2n-SO3H defined as above and where-S- is an atom of bivalent sulphur;
   c) N-alkylaminic-alkylenoxysulphonic (free acid or its alkaline or ammonium salt) or betaine, as non-limiting examples;
   d) -NR"-CnH2n-SO3H, with R" chosen from hydrogen, an alkyl or phenyl group or aromatic alkyl, specifically the group -NR"- is an amino-alcoholic group or ethereal amine or polyaminic group, as may be derived from ethanolamine, N-methyl ethanolamine, amino-propanol (3-amino, or 2-amine), morpholine, 3-methoxyethanolamine, N-tetrahydrofurfurylamine, 6-amino-hexanol, 1-(3-aminopropyl) imidazol, N, N'-dimethyl-ethylenediamine;
      the betainic derivatives containing the groups
   e) -N⁺M (C2H4OH)- CnH2n-SO3⁻ where M is an alkyl;
   f) -N⁺(C2H4OH) 2- CnH2n-SO3⁻;
   g) -N⁺{[(CHOH) 4] CH2OH}- CnH2n-SO3⁻
      all intended as non-limiting examples of the invention.

Specifically, the monovalent perfluorinated radical (Rf-) at the head end is one of the alternative structures of the following type:
C₂F₅-; [CF3-(C2F4)]-; [C2F5-(C2F4)]-; [(CF3) 2CF- (C2F4)]-corresponding to the respective types of telomers initiated by the telogens CF3I, C2F5I, (CF3) 2CFI, (for example illustrated in US 3,156,732, M. Hauptschein, 1964), obtained according to processes known to technicians in the sector, the most common of which uses C2F5I as telogen to obtain a telomer described by the formula [CₖF₂ₖ₊₁]I where k ranges from 2 to 6, preferably from 3 to 6 and even more preferably from 4 to 6; the aliphatic segment (-Rh-), if present, bound to the radical (Rf-) by a C-C bond has a linear or branched structure chosen from the alkylenic group, composed of a number of carbon atoms ranging from 2 to 20, preferably 2 to 10, and even more preferably from 2 to 4.

Among other things the invention lies in the fact that the compound surfactant contains, as well as a hydrophobic head group, polar or polarisable but not ionised hydrophilic groups, as well as the terminal sulphonic group; such hydrophilic groups are situated in an intermediate position between the two terminals of the molecule, that is between the highly hydrophobic and oleophobic perfluorinated head and the dissociated highly hydrophilic and ionic sulphonic tail group.

Said hydrophilic groups, present in the grouping -L-Q-, are of particular significance for application purposes. In fact, the complex molecular structure of the new class of surfactants is characterised by a balancing of hydrophilic-hydrophobic properties which can be varied according to the application. In addition, the presence of non-ionic polar groups or groups which can be polarised introduces interesting properties of anchorage to supports or particles suspended in the solubilisation or dispersion mediums of the surfactant.

More specifically, some of the combinations of the three constituent groups described in the invention, in other words the perfluorinated head group with a highly hydrophobic and oleophobic nature, the other terminal tail group, that is the dissociated and highly hydrophilic ionic sulphonic group, and the intermediate non-ionic polar hydrophilic groups - when present in a lateral position and in the right arrangement - are responsible for the individual physical and chemical properties and also, depending on the ionic characteristics and the pH of the medium, of a surprising inclination towards environmental compatibility, that is to being degradable by physical and organic environmental agents.

One of the key objectives pursued and purpose of the research conducted to achieve the invention was to produce a new series of partially fluorinated surfactants characterised by a structure where the component composed of the perfluorinated segment was limited as far as possible so as to achieve an equilibrated balancing of the perfluorinated head to lower the surface and interfacial tension to below the known limits of conventional hydrocarbon surfactants and to about 30 dine/cm for the surface tension; an important objective of the invention was to limit the length of the perfluorinated segment so as to facilitate its aggression by natural physical and biological environmental agents.

To such purpose it was decided to limit the length of the fluorinated segment (Rf-) to up to 6 atoms of carbon, as well as to pursue a molecular architecture which would facilitate exposure in an aggregate form of the hydro-oleophobic, hydro-fluorocarbon component to the non-polar or gaseous phase, while the hydrophilic components of the two types, the dissociated ionic type of the terminal sulphonic group and that of the polar but not dissociated hydrophilic group formed of polyethereal groups and/or amino groups was given the task of adding other chemical-physical and surface effects.

Specifically, to the non-dissociable polar groups the task is given of conferring a compatibilising agent with the aqueous phase, or for example, having a steric hindrance effect on the dissociated polar group so as to screen and prevent its possible and damaging polarising effect on the erythrocyte cellular membrane, at the moment of the initial action of any organic aggression of living cells by the surfactants; while - as regards the diffusion phase of the surfactant in the atmosphere - the polar hydrophilic groups again have been given the task of favouring compatibility with the environmental substrate and the aggression of the molecular structure of the surfactant by natural physical and chemical-organic agents [cfr. J. G. Riess, Adv. Mater. 3, 249 (1991)].

By choosing the structure and molecular weight of the other structural components of the invention, an attempt was made to optimise the wetting/covering capacity of the final formulations, and to increase the glossiness of the surfaces treated with such formulations.

The chaining of the constituent groups of the structure of the invention was performed by carefully selecting and perfecting the conditions of the methods of synthesis, separation and purification of the products in question and hereafter described.

The raw materials needed to commence the synthesis of the new surfactants described in the invention, composed of the perfluorinated iodotelomers of TFE, were found on the market.

Purely by way of illustration, some examples are given below of the preferred method of synthesis of some embodiments of the invention.

Example 1
Synthesis of

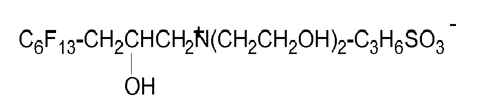

A 500 ml four-necked flask fitted with a mechanical stirrer, a thermometer and condenser dripper fitted to a heat-regulated oil bath, was filled with 15.63 grams (0,0416 moles) of 2,2,3,3,4,4,5,5,6,6,7,7,7-tridecafluoroeptyl) oxyrane, 4.37 grams (0,0416 moles) of diethanolamine and 15.63 grams of 2-propanol in a nitrogen atmosphere.

After 16 hours of reaction at 70°C, and after having verified the disappearance of the reagents by GC analysis, the solvent was distilled at reduced pressure. 20.0 grams of a solid, whitish product were obtained (1).

4.82 grams (0.0395 moles) of 1,3-propansultone were added to the reaction product (1) (0,0402 moles). The reaction was conducted at 100°C for 10 hours.

By means of GC and IR analysis the total disappearance of the sultone was confirmed.

The product was solubilised to 25% in water.
A solution at 0.027% had an interfacial tension of 6.56 mN/m.

Example 2
Synthesis of

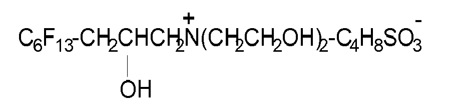

Proceeding as in the previous example the 1,3-propansultone was substituted with 5.38 grams (0.0395 moles) of 1,4-butane sultone. The reaction was performed at the same temperature for a total time of 14.5 hours.

The final product was an amber solid, which was solubilised to 25% in water. A solution at 0.033% had an interfacial tension of 5.58 mN/m.

Example 3
Synthesis of

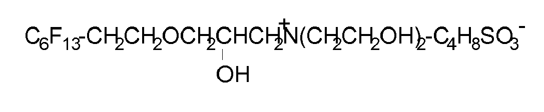

Proceed as in example 1 substituting the 2,2,3,3,4,4,5,5,6,6,7,7,7-tridecafluoroeptyl) oxyrane with 17.47 grams (0.0416 moles) of 3-[2-(perfluoroesil)] ethoxy-1,2-epoxypropane (0.0395 moles) (available from Fluorochem Ltd) and substituting the 1,3-propansultone with 5.38 grams (0.0395 moles) of 1,4-butane sultone. The reaction was performed at the same temperature for a total time of 16 hours.

The product obtained with the butansultone, 5-(NN'-diethanolammonium)-7-hydroxy-9-ossa-tridecafluoro heptadecan -1-sulphonate, betain was:

C6F13-CH2CH2CH2-O-CH2CH(OH)CH2-N(+)[C2HO0H]2-

CH2CH2CH2SO3 (-)

The product was solubilised to 25% in water.

A solution at 0.031% had an interfacial tension of 5.45 mN/m.

Example 4
Synthesis of

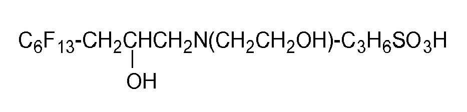

A 50 ml three-necked flask fitted with a mechanical stirrer, a thermometer and condenser dripper fitted to a heat-regulated oil bath, was filled with 5.64 grams (15 moles) of 2,2,3,3,4,4,5,5,6,6,7,7,7-tridecafluoroeptyl)oxyrane, 0.94 grams (15 moles) of ethanolamine and 5.64 grams of 2-propanol in a nitrogen atmosphere.

The reaction was conducted at 70°C for 7 hours. The solvent was distilled, along with any components which did not react, at reduced pressure.

1.73 grams (14.2 moles) of 1,3-propansultone were added to the reaction product (14.6 moles). The reaction was conducted at 70°C for 6 hours.

GC analysis was used to confirm the total disappearance of the sultone.

The product was solubilised to 25% in water.

Example 5
Synthesis of 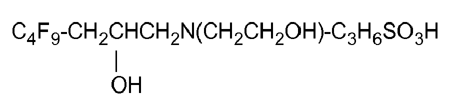 the

Proceed as in example 4 substituting the 2,2,3,3,4,4,5,5,6,6,7,7,7-tridecafluoroeptyl)oxyrane with 4.14 grams (0.15 moles) of 2,2,3,3,4,4,5,5,5-nonafluoropentyl)oxyrane.

Example 6

### Synthesis of C₆F₁₃CH₂CH (OH) CH₂N⁺ (CH₃) (CH₂CH₂OH) -C₃H₆SO₃⁻

A 250 ml four-necked flask fitted with a mechanical stirrer, a thermometer and bubble condenser heated in a heat-regulated oil bath, was filled with 80 grams (0.213 moles) of 2,2,3,3,4,4,5,5,6,6,7,7,7-tridecafluoroeptyl) oxyrane, 15.98 grams (0.213 moles) of 2-methylamino-ethanol and 80 grams of 2-propanol in a nitrogen atmosphere.

The reaction was conducted at 70°C for 7 hours. The solvent was distilled, along with any components which did not react, at reduced pressure.

1.73 grams (14.2 moles) of 1,3-propansultone was added to a part of the reaction product amounting to 14.6 moles. The reaction was conducted at 70°C for 11 hours.

GC analysis was used to confirm the total disappearance of the sultone.

The product was solubilised to 25% in a 50/50 solution of IPA and water.

Example 7

### Synthesis of C₆F₁₃CH₂CH(OH)CH₂N⁺(CH₃) (CH₂CH₂OH)-C₄H₈SO₃

Proceed as in the previous example substituting the 1,3-propansultone with 1.9 grams (0.0142 moles) of 1,4-butane sultone. The reaction was performed at the same temperature for a total time of 14 hours.

The final product was an amber solid.

The product was solubilised to 25% in a 50/50 solution of IPA and water.

Example 8

### Synthesis of C6F13(CH2)11OC4H8SO3H

Preparation of C6F13(CH2)11OH

A 250 ml four-necked Pyrex glass flask, fitted with a mechanical agitator and bubble condenser, heated in an oil bath was filled with 16 grams of undecylenyl alcohol CH2=CH(CH2)9OH (PM 170,29; (0.0939 moles) and 15 grams of an aqueous solution at 35% in weight of sodium metabisulphite. After heating to 80°C while stirring, 75.42 grams of C6F13I were added drop by drop over the space of an hour. Contemporaneously the reactor was filled with 0.08 grams of AIBN in two portions of 0.04 grams each at two-hour intervals.

At the end of the four hours of reaction the procedure continued with rinsing in water, separation and distillation of the solvent and of the remaining reagents. 53.6 grams of product were obtained to which 23.58 grams of ethanol and 11.5 grams of 37% HCl solution were added. The system was heated to 65-70°C and then over two hours 10.52 grams of powdered Zn were added.

At the end of the reaction, by means of distillation 38.53 grams of alcohol C6F13 (CH2) 11OH were obtained.

15 grams of this product (0.0306 moles) were placed in a 100 ml flask containing anhydrous tetrahydrofurane. The process was performed in a nitrogen atmosphere at a temperature of 10 to 15°C.

Under agitation, 0.735 grams of NaH (0.0306 moles) were added over the space of two hours. At the end of this addition, the reaction was continued for another two hours. The reaction was monitored by means of IR analysis until complete disappearance of the oxydrilic group peak at 3400cm-1.

The solvent was distilled at reduced pressure to obtain 14.8 grams (0.028 moles) of a light yellow solid.

Lastly 3.6 grams (0.026 moles) of 1,4-butane sultone and 18.4 grams of anhydrous dioxane were added to this product. The reaction was conducted in a nitrogen atmosphere at 90°C for 7 hours until the complete disappearance of the peak corresponding to the 1,4-butansultone.

The product was solubilised to 25% in a 50/50 mixture of water and isopropanol.

Example 9

The surface tensions of the various surfactants synthesised in the various examples were measured. The concentrations refer to the active matter of the product in demineralised water. Measurement of the surface tension was performed using the Kruss K20 tensiometer.

| EXAMPLE | Percentage concentration (%) | | |
|---|---|---|---|
| | 0.00% | 0.01% | 0.1% |
| 1 | 146,4 | 22,6 | 16,9 |
| 2 | 239,9 | 19,3 | 16,8 |
| 3 | 328,9 | 19,4 | 17,4 |
| 4 | 436,6 | 21,2 | 17,6 |
| 5 | 532,4 | 26,6 | 22,2 |
| 6 | 640,1 | 27,5 | 16,7 |
| 7 | 736,6 | 30,0 | 17,3 |
| 8 | 835,4 | 20,0 | 16,0 |

Example 10

According to a preferred method the 1-iodo-1, 1,2,2-tetrahydrononafluorohexane was subjected to an exchange reaction with dietanolamine, obtaining the corresponding dietanolamine nonafluorohexane. The butansultone was added to such compound in THF and the desired 5-(N, N'-diethanol ammonium) tridecafluoro tridecan -1-sulphonate, betain (3 b) was obtained.

C4F9-CH2CH2-N (+) [C2H4OH] 2 -CH2CH2CH2CH2SO3 (-)

Example 11

According to another method the tridecafluorononiloxyrane was made to react with (D)-glucamine [1-amino-1-deoxy sorbitol ] NH2-[CH(OH)]4 - CH2OH, prepared according to the description in J. Am. Chem. Soc. 72, 5416 (1950)to obtain the following product:

C6F13-CH2-CH(OH)-CH2-NH -[ CH (OH) ]4-CH2OH

Such compound was made to react with the propansultone to obtain the final surfactant:

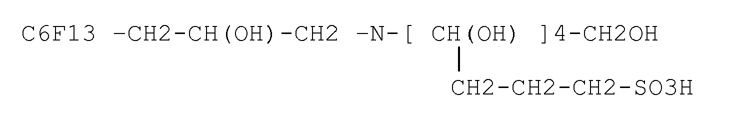

Example 12

A proteic concentrate was prepared destined for use in extinguishing foams according to the following formula:
- proteic concentrate at 53%: 100 grams;
- water: 27 grams;
- 2-propanol: 7 grams;
- dodecylbenzenesulphonatesodium at 30%: 9.5 grams;
- monolaurate sorbitan(HLB 8.6): 4 grams;
- fluorinated surfactant at 25%: 5 grams.

6 grams of such concentrate were taken and diluted with 94 grams of deionised water.

The characteristics of the foam were determined by means of a series of simple tests making it possible to ascertain the effect of the addition of the fluorinated surfactant.

Expansion rate ER

100 ml of diluted solution was transformed into foam using a two whisk mechanical stirrer. The rate of expansion ER is defined as the TE=V/v ratio where V indicates the volume of the final foam and v the volume of the initial solution completely transformed into foam.

Decanting time DT

The time required to collect a volume of liquid corresponding to a quarter of the volume of solution transformed into foam, that is 25 ml, into a graduated cylinder 25 ml.

Foaming power FP

This is the volume of foam formed by pouring 500 ml of foaming solution from a dripper funnel with an outlet diameter of 13 mm placed at a height of 450 mm, onto 100 ml of the same solution contained in a 2-litre container. The volume of the foam is measured 30 seconds after the introduction of the 500 ml of solution.

Fluidity of the foam FF

The fluidity of the foam is determined by measuring the speed at which the foam slides along a plane inclined at 10°. Such inclined plane is the bottom of a containment tray of the foam 35 cm long, 21 cm wide and 10 cm high. Such portion of the tray is separated from a second container by a moving panel. The latter container contains two litres of foam. From the moment in which the moving panel is raised the time which it takes the foam to cover the entire inclined plane of the tray is measured.

The results of the aforesaid tests are shown in the following table:

| | Product concentrate Example 2 | Product concentrate Example 4 |
|---|---|---|
| TE | 9 | 9 |
| TD (sec) | 350 | 360 |
| PS (ml) | 320 | 350 |
| FS (sec) | 12 | 11 |

A person skilled in the art may make modifications to the embodiments described above so as to satisfy contingent requirements while remaining within the scope of protection of the following claims.

Each of the characteristics described as pertaining to one possible embodiment is safeguarded independently of the other embodiments described.

## Claims

1. A sulphonic function compound of the following general formula (I):
R'-L-Q-S (I)
wherein:
- R' is a perfluorinated radical Rf or an Rf-Rh radical wherein Rf is a segment of perfluorocarbon chain composed of 2 to 6 atoms of carbon, Rh is an alkyl or alkenylic, linear or branched, group composed of a number of carbon atoms ranging from 2 to 20;
- L is a bivalent radical, for example 1-oxy-2-hydroxypropylene, 2-hydroxypropylene, oxyalkylene, oxyethylene, oxybutylene, oxy-poly (ethylenoxy); a polyethereal segment (C2H4-O)p-(C3H6O)q-, wherein p is a whole number ranging from 1 to 10 and q is a whole number ranging from 0 to 6;
- Q-S is a radical chosen from the group comprising:
a) -O-CnH2n-SO3H, wherein the sulphonic group is present in the acid or alkaline salt form, the group -CnH2n- is a linear or branched alkylene, wherein n is a whole number ranging from 2 to 8;
b) -S-CnH2n-SO3H, wherein -CnH2n- is defined as above;
a grouping of the alkylamino alkylene sulphonic or alkylammonium alkylensulphonic type such as the groups:
c) -NR"-CnH2n-SO3H, wherein R" is chosen from hydrogen, an alkyl or phenyl group or alkyl-aromatic;
d) -N⁺M(C2H4OH)-CnH2n-SO3⁻, wherein CnH2n is defined as above and M is an alkyl;
e) -N⁺(C2H4OH)2-CnH2n-SO3⁻, wherein CnH2n is defined as above; or
f) -N⁺{[(CHOH)4]CH2OH}-CnH2n-SO3⁻, wherein CnH2n is defined as above.

2. Compound according to claim 1, wherein Rh is a group composed of a number of carbon atoms ranging from 2 to 10.

3. Compound according to claim 1 or 2, wherein Rh is a group composed of a number of carbon atoms ranging from 2 to 4.

4. Compound according to any of the previous claims, wherein p is a whole number ranging from 1 to 6.

5. Compound according to any of the previous claims, wherein n is a whole number ranging from 3 to 4.

6. Compound according to any of the previous claims, wherein the group -NR"- is an amino-alcoholic or amino-ether group or a polyaminic group, for example obtainable from ethanolamine, N-methyl ethanolamine, 3-amino-propanol, 2-amino-propanol, morpholine, 3-methoxyethanolamine, N-tetrahydrofurfuryl-amine, 6-aminohexanol, 1-(3-aminopropyl)imidazol, N,N'-dimethylethylenediamine.

7. Compound according to any of the previous claims, wherein Rf is a radical of the formula (CₖF₂ₖ₊₁)-, wherein k is a whole number ranging from 2 to 6, preferably from 4 to 6.

8. Method for the synthesis of a compound having a general formula (I) according to any of the previous claims.

9. Use of the compound having the general formula (I) according to any of the claims from 1 to 7, pure or mixed, as a surfactant or synergic agent able to reduce the surface tension of aqueous compositions to below 30 mN/m.

10. Use of the compound having the general formula (I) according to any of the claims from 1 to 7, pure or mixed, to lower the surface tension of fluid or solid materials to which it is applied.

11. Use of the compound having the general formula (I) according to any of the claims from 1 to 7, pure or mixed, as an additive for emulsion compositions, as an additive for varnishes, as an additive in processes for treating paper and fabrics, as an additive in enamels and waxes for floors or furniture or for shoes, as an additive in cleaning products, as an additive in formulations to prevent glass from fogging, as an additive to aqueous extinguishing compositions, and/or as an additive to improve the glossiness of surfaces.

12. Use of the compound having the general formula (I) according to any of the claims from 1 to 7, pure or mixed, as a corrosion inhibitor.

13. Use of the compound having the general formula (I) according to any of the claims from 1 to 7, pure or mixed, as a wetting agent.

14. Use of the compound having the general formula (I) according to any of the claims from 1 to 7, as an additive to paints in quantities ranging from 0.001% to 10% in weight.

15. Use of the compound having the general formula (I) according to any of the claims from 1 to 7, as an additive for aqueous extinguishing compositions, in quantities ranging from 0.001% to 10% in weight.
